# EUROPEAN PATENT APPLICATION

(11) **EP 2 156 827 A1**
(43) Date of publication of application: **24.02.2010**
(21) Application number: 08721409.4
(22) Date of filing: 05.03.2008
(51) Int. Cl.: A61K 9/70, A61L 15/00

(54) **POULTICE AND PROCESS FOR PRODUCING THE POULTICE**

(30) Priority: 17.05.2007 JP 2007132218
(71) Applicant: NIPRO Patch Co., Ltd., Saitama 344-0057 (JP)
(72) Inventor: NOGUCHI, Kenichi, Kasukabe-shi Saitama 344-0057 (JP)
(74) Representative: Maggs, Michael Norman
(86) International application number: PCT/JP2008/053989
(87) International publication number: WO 2008/142896

(57) **Abstract**

This invention provides a poultice, which can prevent both runover of a medicated layer and separation from a subject for which the poultice is applied, and a process for producing the poultice. A poultice (10) comprises a support (20) and a medicated layer (30) provided on the support (20) and formed of a medicine. The medicated layer (30) comprises edge parts (31) which are a part extended from the outer periphery inward by not more than 5 mm, and a central part (33) held between the edge parts (31). The mass of the medicine per unit area of the whole medicated layer (30) is not more than 0.10 g/cm², and the mass of the medicine per unit area in the edge parts (31) is not less than 70% of the mass of the medicine per unit area in the central part (33).

## Description

### TECHNICAL FIELD

The present invention relates to a poultice, and a process for producing the poultice.

### BACKGROUND ART

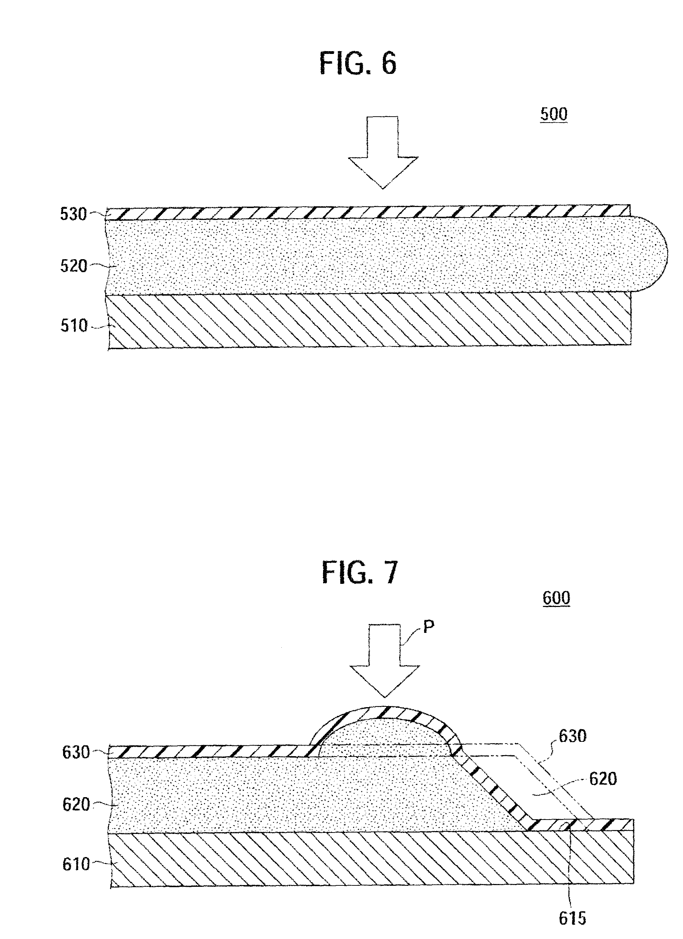

Conventionally, poultices containing a variety of drug ingredients have been developed and commercialized. Fig. 6 shows a cross-sectional view illustrating a marginal area of a poultice 500 according to a prior art example. As shown in Fig. 6, a conventional poultice 500 has a backing 510 formed of a nonwoven fabric or the like, and a paste layer 520 provided on the backing 510, in which a drug ingredient is included in the paste layer 520.

Such poultice 500 has been distributed in a state of being stacked in numerous numbers after the paste layer 520 was covered with a release sheet 530. Thus, a significant pressure P may be loaded on each poultice 500, whereby the paste layer 520 may run over the outer periphery of the poultice 500. In such a case, since the runover of the paste layer 520 may adhere to the user and the like, inferior handling characteristics of the poultice 500 are inevitable.

Fig. 7 shows a cross-sectional view illustrating a marginal area of a poultice 600 according to other prior art example. In view of the foregoing problems, the poultice 600 disclosed in Patent Document 1 is provided with an unformed part 615 of a paste layer 620 on the end of a backing 610. According to the poultice 600, the paste layer 620 squashed due to the load of the pressure P is retained in the unformed part 615; therefore, the runover of the paste layer 620 from the outer periphery of the poultice 600 can be prevented.

Patent Document 1: Japanese Unexamined Patent Application, First Publication No. Hei 9-110679

### DISCLOSURE OF THE INVENTION

### Problems to be Solved by the Invention

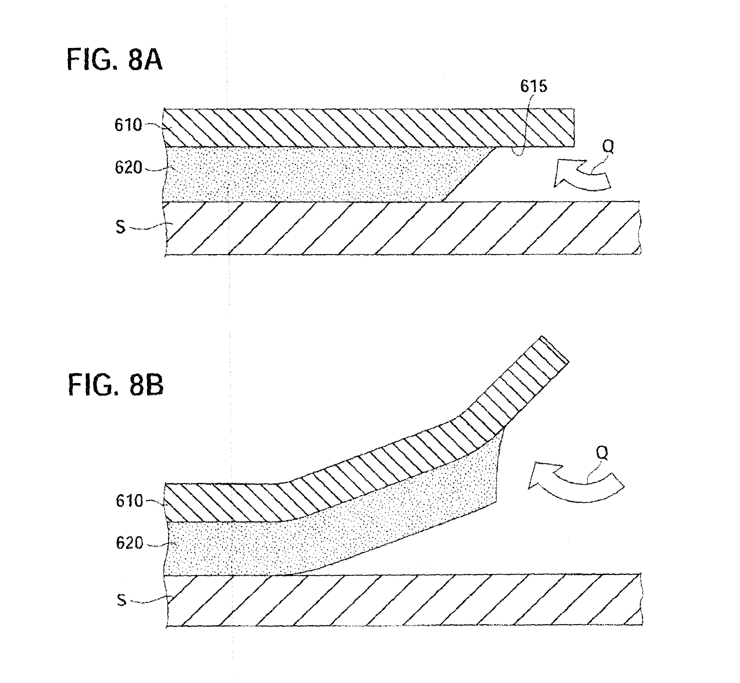

However, since the paste layer 620 is not present in the unformed part 615 according to the poultice 600 described above, the unformed part 615 are not in contact with the user's skin S in use (see Fig. 8 (A)). Thus, when an external force Q is applied to the poultice 600 by friction or the like, the poultice 600 is easily detached from the skin S at the unformed part 615 that serves as an initiation site (see Fig. 8 (B)). Such a problem is particularly critical when the poultice is in use for a long time period, for example, during time of sleep.

The present invention was made in view of the foregoing circumstances, and an object thereof is to provide a poultice, which can prevent both runover of a paste layer and detachment from a portion to which the poultice is applied, and a process for producing the poultice.

### Means for Solving the Problems

The present inventors found that both runover of a paste layer and detachment from a portion to which the poultice is applied can be prevented by placing a paste over the entirety of the backing approximately evenly in small quantities, thereby achieving the present invention. Specifically, the present invention provides aspects as in the following.

According to a first aspect of the present invention, a poultice includes a backing, and a paste layer, formed of a paste, provided on the backing, in which:
the paste layer includes an edge part extending from an outer periphery inwardly by no more than 5 mm, and a central part interposed between the edge part;
a mass of the paste per unit area of the paste layer as a whole is no more than 0.10 g/cm²; and
a mass of the paste per unit area in the edge part is no less than 70% of a mass of the paste per unit area in the central part.

When the mass of the paste in the edge part is too low as compared with the mass of the paste in the central part, an excessively large difference between the thickness of the edge part and that of the central part results. Therefore, it is likely to fall in the situation in use in which the central part is in contact with the portion of application, while the edge part are not in contact with the portion.

Thus, according to the first aspect of the present invention, the mass of the paste per unit area in the edge part is defined to be no less than 70% of the mass of the paste per unit area in the central part, whereby the difference between the thickness of the edge part and that of the central part falls within a permissible range, and the situation in which the edge part is not in contact with the portion of application in use can be prevented.

On the other hand, due to a small difference between the thickness of the edge part and that of the central part, permissible level of retainable amount of the paste that moves from the central part to the edge part when the paste layer is squashed in the process of distribution and the like is limited. When the paste in an amount beyond the permissible level is moved, runover of the paste layer from the outer periphery of the poultice results.

Thus, according to the first aspect of the present invention, since the mass of the paste per unit area of the paste layer as a whole is defined in the range of necessary and sufficient mass, i.e., no more than 0.10 g/cm², detachment from a portion of application due to insufficient adhesion can be prevented, while runover of the paste layer can be prevented.

According to a second aspect of the poultice as described in the first aspect of the present invention, the paste contains a compound represented by formula 1, and does not substantially contain an adhesion-enhancing component that enhances adhesion. 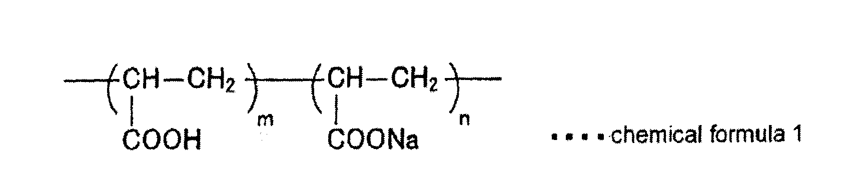 Where, m/n (molar ratio) is no less than 55/45 and no greater than 75/25.

Since the mass per unit area of the paste that constitutes the paste layer is comparatively low, it is probable that the poultice may detach from a portion of application due to insufficient adhesion of the paste.

Thus, according to the second aspect of the present invention, a copolymer having a ratio of an included acrylic acid to sodium acrylate is no less than 55/45 (molar ratio) is added to the paste layer, whereby the adhesion is improved sufficiently, and detachment of the poultice from the portion of application over time can be prevented. In this respect, a copolymer of acrylic acid and sodium acrylate may lead to insufficient adhesion to the portion of application when the ratio of the included acrylic acid to sodium acrylate is too low (for example, m/n (molar ratio) of 50/50).

In addition, since the adhesion is satisfactorily improved, detachment of the poultice from the portion of application over time can be prevented even though the paste layer does not substantially contain an adhesion-enhancing component.

Thus, according to the second aspect of the present invention, the bulkiness of the paste layer can be reduced since the paste layer does not substantially contain an adhesion-enhancing component such as polybutene, an alicyclic saturated hydrocarbon resin, a rhodine derivative, a terpene based resin, a phenol based resin or the like. Accordingly, adhesiveness to the portion of application, and capability of conforming to an altered shape etc., of the portion of application can be improved. In addition, since the amount of the added adhesion-enhancing component is significantly reduced, the poultice can be produced at a low cost. Furthermore, when the step of adding the adhesion-enhancing component is omitted, the production steps are simplified, whereby the poultice can be produced easily at a lower cost.

Moreover, when the ratio of the included acrylic acid to sodium acrylate is too high (for example, m/n (molar ratio) of 80/20), production of the copolymer may be technically difficult, whereby the yield can be lowered.

Thus, according to the second aspect of the present invention, since a copolymer having a ratio of the included acrylic acid to sodium acrylate of no greater than 75/25 (molar ratio) is added to the paste layer, the copolymer can be produced easily with a high yield.

The term "adhesion-enhancing component" as used herein means a component added for the purpose of enhancing adhesion, and any component which is added for other purpose but may increase adhesion (for example, a binding component such as gelatin, and a wetting component such as glycerin) is excluded.

Further, the phrase "not substantially contain" refers to a state of not containing an amount in a range that can achieve promotion of adhesion.

The designation "m/n" in formula 1 means a ratio of the included acrylic acid to sodium acrylate in the entire copolymer of acrylic acid and sodium acrylate contained in the paste layer. Accordingly, the copolymer added to the paste layer may be one, or two or more.

According to a third aspect of the poultice as described in the second aspect of the present invention, the adhesion-enhancing component is at least one selected from the group consisting of polybutene, an alicyclic saturated hydrocarbon resin, a rhodine derivative, a terpene based resin and a phenol based resin.

According to a fourth aspect of the poultice as described in either of the second or third aspect of the present invention, the paste layer does not substantially contain a plasticizer of the adhesion-enhancing component.

According to the fourth aspect of the present invention, the poultice can be produced at a low cost since the paste layer does not substantially contain a plasticizer such as liquid paraffin. In addition, when the step of adding a plasticizer is omitted, the production steps can be further simplified, whereby the poultice can be produced more easily at a lower cost.

Since the paste layer does not substantially contain the adhesion-enhancing component, the poultice is not rigidified even though a plasticizer is not added to the paste layer. Thus, capability of conforming to the portion of application can be maintained.

According to a fifth aspect of the poultice as described in the fourth aspect of the present invention, the plasticizer is liquid paraffin.

According to a sixth aspect of the poultice as described in any one of the second to fifth aspects of the present invention, the paste layer has a pH of no lower than 4.50 and no higher than 5.50.

According to the sixth aspect of the present invention, since the paste layer has a pH of no lower than 4.50 and no higher than 5.50, the adhesion can be further improved, and residue of the paste left behind after peeling off from the portion of application can be further prevented.

According to a seventh aspect of the poultice as described in any one of the first to sixth aspects of the present invention, the backing has a mass per unit area of no greater than 100 g/m².

When the mass per unit area of the backing is too low, it is probable that the paste may pass through the nonwoven fabric to exudate therefrom, while when the mass per unit area is too high, the flexibility may be reduced, whereby capability of conforming to an altered shape etc., of the portion of application may deteriorate.

In this respect, since the paste layer in the present invention has a paste in an amount of as small as no more than 0.10 g/cm², occurrence of an event such as exudation of the paste can be prevented. Thus, according to the seventh aspect of the present invention, since the mass per unit area is defined to a low level of no more than 100 g/m², exudation of the paste can be prevented while exhibiting superior flexibility and being able to improve capability of conforming to an altered shape etc., of the portion of application.

According to an eighth aspect of the present invention, a process for producing a poultice, which includes a backing and a paste layer, formed of a paste, provided on the backing,
includes a pressing step of pressing the paste layer at sites to be a peripheral margin to form an edge part and a central part interposed between the edge part, in which a thickness of the edge part is less than a thickness of the central parts, and
in which the pressing step includes a regulating step of regulating a force of the pressing, thereby making a difference between the thickness of the edge part and the thickness of the central part to be no greater than a predetermined value.

According to the eighth aspect of the present invention, an edge part and a central part interposed between the edge part is formed, in which a thickness of the edge part is less than a thickness of the central part. Thus, when the paste layer is squashed in the distribution process and the like, the paste moved from the central part is retained to meet the difference in the thickness, whereby runover of the paste layer from the outer periphery of the poultice is prevented.

In addition, since the pressing step includes a regulating step, the difference between the thickness of the edge part and that of the central part falls within the range of no greater than a predetermined value, and thus the situation in which the edge part is not in contact with the portion of application when in use can be prevented.

Therefore, both runover of a paste layer and detachment from a portion to which the poultice is applied can be prevented.

### Effects of the Invention

According to the present invention, since the mass of the paste per unit area in the edge part is defined to be no less than 70% of the mass of the paste per unit area in the central part, the difference between the thickness of the edge part and that of the central part falls within a permissible range, and thus the situation in which the edge part is not in contact with the portion of application in use can be prevented.

In addition, since the mass of the paste per unit area of the paste layer as a whole is defined in the range of necessary and sufficient value, i.e., no more than 0.10 g/cm², detachment from a portion of application due to insufficient adhesion can be prevented, while enabling runover of the paste layer to be prevented.

### BRIEF DESCRIPTION OF THE DRAWINGS

Fig. 1 shows a partial cross-sectional view illustrating a poultice according to one embodiment of the present invention;
Fig. 2 shows a view illustrating a process for producing the poultice shown in Fig. 1;
Fig. 3 shows a plan view illustrating the poultice shown in Fig. 1;

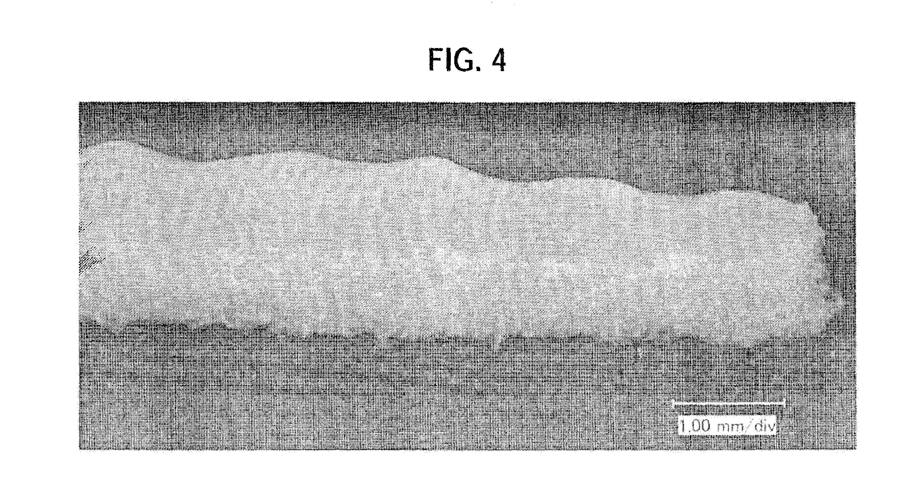

Fig. 4 shows a photograph of a partial cross section of a poultice according to one Example of the present invention;

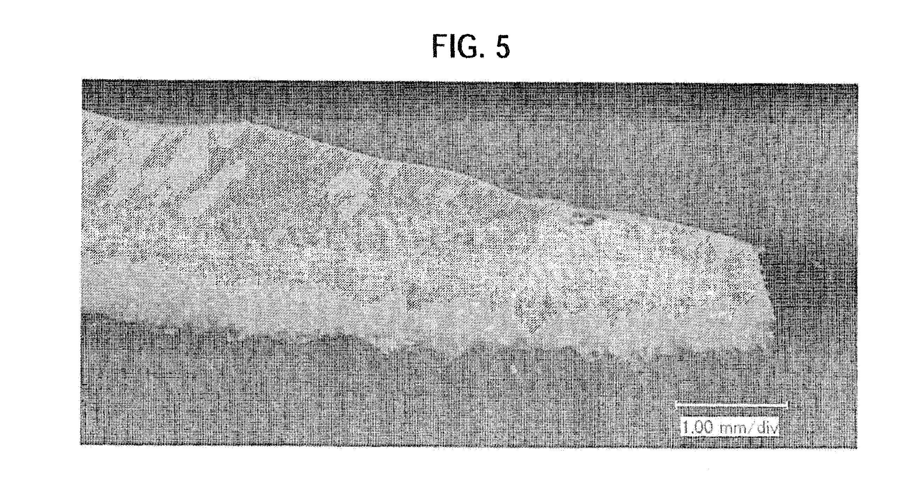

Fig. 5 shows a photograph of a partial cross section of a poultice according to a prior art example;
Fig. 6 shows a partial cross-sectional view illustrating the poultice according to the prior art example;
Fig. 7 shows a partial cross-sectional view illustrating a poultice according to another prior art example; and
Fig. 8 shows a view illustrating the state of use of the poultice shown in Fig. 7.

### EXPLANATION OF REFERENCE NUMERALS

- 10: poultice
- 20: backing
- 30: paste layer
- 31: edge part
- 33: central part
- 40: release sheet

### PREFERRED MODE FOR CARRYING OUT THE INVENTION

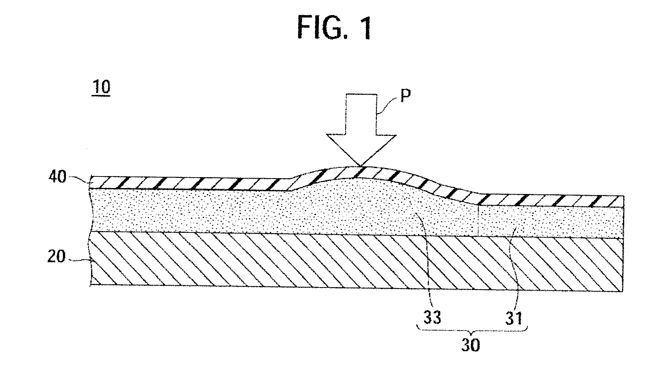

One of the embodiments of the present invention will be explained below with reference to the drawing. Fig. 1 shows a cross-sectional view illustrating the end of a poultice 10 according to one embodiment of the present invention.

The poultice 10 includes a backing 20, and a paste layer 30 provided on the backing 20. In addition, the poultice 10 further includes a release sheet 40 that covers the paste layer 30 in Fig. 1. Each of the constitutive elements is explained below in detail.

### Backing

The backing is not particularly limited as long as it can support the paste layer. A stretch or nonstretch backing can be used, and specifically, a fiber sheet, a resin film or the like can be exemplified. Of these, in view of capability of preventing skin irritation or steaminess resulting from sweat or the like, fiber sheets constituted with a woven fabric or nonwoven fabric having water vapor transmittivity are preferred.

More specifically, synthetic fibers or natural fibers such as polyurethane, polyester, polypropylene, vinyl polyacetate, polyvinylidene chloride, polyethylene, polyethylene terephthalate, aluminum sheets, nylon, acryl, cotton, rayon and acetate, or fiber sheets of a woven fabric or nonwoven fabric produced using these fibers in combination may be exemplified. Furthermore, fiber sheets constituted with a composite material etc., of any of these and a film having water vapor transmittivity may be exemplified.

Among these, in light of safety, versatility and stretchability, fiber sheets of a woven fabric or nonwoven fabric constituted with polyester or polypropylene are preferred, and fiber sheets of a nonwoven fabric constituted with polyester or polypropylene are more preferred. Since such fiber sheets are superior in softness, they can follow the movement of the portion of application, accompanied by less irritation to skin. Additionally, when such a fiber sheet is used, a skin patch having an appropriate self-supporting property can be obtained.

When the mass per unit area of the backing is too low, the strength thereof may be insufficient. To the contrary, when the mass per unit area is too high, a significant uncomfortable feeling may be experienced by the portion of application. Accordingly, the mass per unit area of the backing is preferably no less than 70 g/m² and no more than 100 g/m², and more preferably no less than 80 g/m² and no more than 90 g/m².

### Paste Layer

The paste layer 30 is formed of a paste, and is a part to be in contact with the portion of application (for example, user's skin) when in use. Such a paste layer 30 includes an edge part 31 described later, and a central part 33 interposed between the edge part 31.

The present invention is characterized by the mass of the laminated paste. More specifically, the mass of the paste per unit area of the paste layer 30 as a whole is no more than 0.10 g/cm², and the mass of the paste per unit area in the edge part 31 is no less than 70% of the mass of the paste per unit area in the central part 33. In view of capability of preventing the runover, the mass of the paste per unit area in the edge part 31 is preferably no more than 110% of the mass of the paste per unit area in the central part 33.

The mass of the paste per unit area of the paste layer 30 as a whole is preferably no more than 0.07 g/cm² in view of capability of further preventing the runover of the paste layer. On the other hand, the mass of the paste per unit area of the paste layer 30 as a whole is preferably no less than 0.05 g/cm² in view of capability of preventing detachment from a portion of application due to insufficient adhesion. Therefore, the mass of the paste per unit area of the paste layer 30 as a whole is most preferably about 0.05 g/cm².

The mass of the paste per unit area of the paste layer 30 as a whole correlates with the ratio of the mass of the paste per unit area of the edge part 31 to that of the central part 33. More specifically, as the mass of the paste per unit area of the paste layer 30 as a whole becomes lower, a permissible range of the ratio of the mass of the paste per unit area of the edge part 31 to that of the central part 33 widens. Specifically, when the mass of the paste per unit area of the paste layer 30 as a whole is 0.07 g/cm², the mass of the paste per unit area in the edge part 31 is preferably no more than 120% of the mass of the paste per unit area in the central part 33; when the mass of the paste per unit area of the paste layer 30 as a whole is 0.05 g/cm², the mass of the paste per unit area in the edge part 31 is preferably no more than 150% of the mass of the paste per unit area in the central part 33; and when the mass of the paste per unit area of the paste layer 30 as a whole is 0.035 g/cm², the mass of the paste per unit area in the edge part 31 is preferably no more than 200% of the mass of the paste per unit area in the central part 33. The foregoing description means that the likelihood of generating defective products producing runover of the paste layer can be reduced by lowering the mass of the paste per unit area of the paste layer as a whole.

When the mass of the paste per unit area of the paste layer 30 as a whole is no more than 0.07 g/cm², the mass of the paste per unit area in the edge part 31 may be generally no less than 70% and no more than 150% of the mass of the paste per unit area in the central part 33, preferably no less than 75% and no more than 130%, more preferably no less than 80% and no more than 120%, and most preferably no less than 90% and no more than 110%.

Such a paste may be constituted with a conventionally well-known composition. However, the composition as described below is preferred since sufficient adhesion can be achieved with a lower mass of the paste.

### Water Soluble Polymer

To the paste layer in the poultice of the present invention is added a copolymer of acrylic acid and sodium acrylate, as a water soluble polymer. In the copolymer, the ratio of the included acrylic acid to sodium acrylate is no less than 55/45 and no greater than 75/25 (molar ratio). As such a copolymer, for example, "NP-800 (registered trademark)" (manufactured by Showa Denko K.K.) may be employed.

In view of the capability of improving adhesion and preventing residue of the paste after peeling off from the portion of application, the content of the copolymer is preferably no lower than 1% by mass and no higher than 10% by mass with respect to the poultice as a whole. The content of the copolymer is more preferably no lower than 3% by mass and no higher than 9% by mass, and most preferably no lower than 5% by mass and no higher than 8% by mass. It should be noted that the content of the copolymer may be predetermined appropriately taking into account the adhesive property, agglutinative property, shape retainability, water absorptivity of the poultice, uniformity of the paste, processing characteristics, feel in use, and the like. pH

The pH of the paste layer is generally adjusted to about 6 to 7 for reducing irritation to the portion of application. In the present invention, further taking into account the capability of further improving the adhesion, the pH is preferably no lower than 4.50 and no higher than 5.50. The pH is more preferably, no lower than 4.60 and no higher than 5.20.

With regard to the relationship between the pH and improvement of adhesion, the following mechanism may be assumed. Lowering of the pH leads to an increase in the ratio of acrylic acid to sodium acrylate included in the paste layer, whereby the adhesion may be improved.

In addition, the poultice may contain at least one water soluble polymer such as gelatin, polyvinyl alcohol, polyacrylamide, polyethyleneoxide, polyethyleneimine, polyvinylpyrrolidone, carboxyvinyl polymer, methyl cellulose, carboxymethyl cellulose, hydroxyethyl cellulose, and the like. Water

Examples of water added to the paste layer include purified water, sterile water, natural water and the like. Water serves as a dispersant or a solubilizer of the water soluble polymer and the like, and plays an important role in uniformly dispersing and dissolving glycols and polyhydric alcohol which may be added as a moisturizing agent described later. Moreover, water may improve the feel in use, and transfers to the skin of the portion of application, thereby providing moisture and suppleness.

When the content of water is too low, the workability may deteriorate, or the production cost may increase. On the other hand, when the content is too high, the shape retainability may deteriorate. Thus, the content of water is preferably no lower than 30% by mass and no higher than 95% by mass based on the paste layer as a whole. The content of water is more preferably no lower than 35% by mass and no higher than 80% by mass, and most preferably no lower than 40% by mass and no higher than 60% by mass. The content of water may be predetermined appropriately taking into account the adhesive property, water holding capacity, workability, shape retainability etc., of the formulation.

### Optional Ingredient

### Polyhydric Alcohol

Examples of polyhydric alcohol which may be added to the paste layer include glycerin, ethylene glycol, 1,3-butylene glycol, propylene glycol, dipropylene glycol, sorbitol, and xylitol. Among chem, glycerin is preferred in view of capability of improving the workability, the feel in use and the like.

When the content of the polyhydric alcohol is too low, the moisture in the poultice volatilizes resulting in a reduction of the adhesion, whereby detachment is likely to occur. On the other hand, too high content of the polyhydric alcohol leads to hardening of the paste, and thus the production may be difficult. The content of the polyhydric alcohol is preferably no lower than 10% by mass and no higher than 50% by mass based on the paste layer as a whole. The content of the polyhydric alcohol is more preferably no lower than 15% by mass and no higher than 45% by mass, and most preferably no lower than 20% by mass and no higher than 40% by mass.

### Polyvalent Metal Salt

Examples of the polyvalent metal salt include aluminum hydroxide, aluminum hydroxide gel, hydrated aluminum silicate, synthetic aluminum silicate, kaolin, aluminum acetate, aluminum lactate, aluminum stearate, calcium chloride, magnesium chloride, aluminum chloride, magnesium aluminometasilicate, and magnesium aluminosilicate. Among these, synthetic aluminum silicate and magnesium aluminometasilicate are preferred.

When the content of the polyvalent metal salt is too low, the degree of crosslinking may be insufficient, leading to deficiency in gel strength. On the other hand, when the content of the polyvalent metal salt is too high, the reaction velocity in production may be excessively accelerated to result in nonuniform gelation, and thus the workability is likely to be unsatisfactory. Accordingly, the content of the polyvalent metal salt is preferably no lower than 0.01% by mass and no higher than 5% by mass based on the paste layer as a whole. The content of the polyvalent metal salt is more preferably no lower than 0.02% by mass and no higher than 3% by mass, and most preferably no lower than 0.03% by mass and no higher than 2% by mass.

To the paste layer may be added as a gelation speed regulating agent, an organic acid such as EDTA, acetic acid, lactic acid, oxalic acid, citric acid or tartaric acid, or an organic acid salt such as sodium EDTA-2, calcium citrate, sodium citrate or disodium citrate, having a chelating action on metal ions.

### Surfactant

Examples of the surfactant which may be added include nonionic surfactants such as sodium dioctylsulfosuccinate, alkyl sulfate salts, 2-ethylhexylalkylsulfuric acid ester sodium salts, and sodium normal dodecylbenzene sulfonate; cationic surfactants such as hexadecyltrimethylammonium chloride, octadecyldimethylbenzylammonium chloride, and polyoxyethylene dodecylmonomethylammonium chloride; and nonionic surfactants such as polyoxyethylene stearyl ether, polyoxyethylene hydrogenated castor oil, polyoxyethylene tridecyl ether, polyoxyethylene nonylphenyl ether, polyoxyethylene octylphenyl ether, polyoxyethylene monostearate, sorbitan monostearate, sorbitan monopalminate, sorbitan sesquioleate, polyoxyethylene sorbitan monolaurate, polyoxyethylene sorbitan monooleate, glycerol monostearate, polyglycerin fatty acid esters, and polyoxyethylene octadecylamine.

When the content of the surfactant is too low, bleeding of components is likely to occur. On the other hand, when the content of the surfactant is too high, shape retainability may be unsatisfactory. Accordingly, the content of the surfactant is preferably no lower than 0.01% by mass and no higher than 5% by mass based on the paste layer as a whole. The content of the surfactant is more preferably no lower than 0.05% by mass and no higher than 4% by mass, and most preferably no lower than 0.1% by mass and no higher than 3% by mass.

### Others

Furthermore, to the paste layer of the present invention may be added an antioxidant, a crosslinking agent, a drug, an antiseptic agent, a tackifier, a solubilizer, a dye, fragrance, an ultraviolet ray absorbing agent, an inorganic filler, a pH adjusting agent, and the like.

### Antioxidant

Examples of the antioxidant include ascorbic acid, propyl gallate, butylhydroxyanisole, dibutylhydroxytoluene, nordihydroguaiaretinoic acid, tocopherol, tocopherol acetate, natural vitamin E, and the like.

### Crosslinking Agent

Examples of the crosslinking agent include thermosetting resins such as water insoluble aluminum compounds, polyfunctional epoxy compounds, amino resins, phenol resins, epoxy resins, alkyd resins and unsaturated polyesters, and inorganic crosslinking agents such as isocyanate compounds, block isocyanate compounds, organic crosslinking agents and metals or metal compounds, which may be used alone or in combination.

### Drug

The drug is not particularly limited as long as it has a pharmacological activity, and examples thereof include antiphlogistic analgetic agents (indomethacin, ketoprofen, flurbiprofen, felbinac, ketorolac, diclofenac, loxoprofen, or salts thereof etc.), antiemetic drugs (granisetron hydrochloride, azasetron hydrochloride, ondansetron hydrochloride, ramosetron hydrochloride etc.), hormone drugs (estradiol, estrone, estriol, equilin, equilenin, progesterone, hydroxyprogesterone caproate, medroxyprogesterone acetate, dydrogesterone, chlormadinone acetate, ethisterone, dimethisterone, norethisterone, norethisterone acetate, norethisterone enanthate, ethynodiol acetate, megestrol acetate, allyl estrenol etc.), frequent urination agents (oxybutynin hydrochloride), calcium antagonists (nifedipine, nisoldipine, nicardipine, nitrendipine etc.), corticosteroids (hydrocortisone, prednisolone, clobetasol propionate etc.), hypnotic sedative agents (phenobarbital, triazoram, nitrazepam, lorazepam etc.), tranquilizers (fluphenazine, diazepam, chlorpromazine etc.), antihypertensive agents (clonidine, clonidine hydrochloride, pindolol, propranolol, nitrendipine, metoprolol etc.), hypotensive diuretic agents (hydrothiazide etc.), antibiotics (penicillin, tetracycline, erythromycin, chloramphenicol etc.), anesthetic agents (lidocaine, dibucaine hydrochloride, ethyl aminobenzoate etc.), antibacterial agents (benzalkonium hydrochloride, clotrimazole etc.), vitamin preparations (vitamin A etc.), antiepileptic agents (nitrazepam etc.), coronary vasodilator agents (nitroglycerin, isosorbide nitrate etc.), antihistaminic agents (diphenhydramine, chlorpheniramine etc.), antitussive agents (tulobuterol hydrochloride, salbutamol, ketotifen fumarate, tranilast, isoproterenol hydrochloride etc.), antidepressant agents (clomipramine hydrochloride, amitriptyline hydrochloride etc.), cerebral circulation activating agents (dihydroergotoxin mesilate, ifenprodil etc.), antitumor agents (5-fluorouracil etc.), muscle relaxing agents (eperisone, dantrolene etc.), analgesic agents (fentanyl, morphine etc.), polypeptide hormone preparations (luteinizing hormone releasing hormone (LH-RH), thyrotropin releasing hormone (TRH) etc.), peripheral vasodilating agents, immunomodulatory agents (polysaccharides, auranofin, lobenzarit etc.), choleretic agents (ursodesoxycholic acid etc.), diuretic agents (hydroflumethiazide etc.), diabetes remedy agents (tolbutamide etc.), therapeutic agents for gout (colchicine etc.), antiparkinson agents (amantadine, levodopa etc.), antivertiginous agents (difenidol, betahistine etc.), and the like.

### Antiseptic Agent

Examples of the antiseptic agent include ethyl paraoxybenzoate, propyl paraoxybenzoate, and butyl paraoxybenzoate.

### Shape Retaining Agent

Examples of the shape retaining agent include casein, pullulan, agar, dextran, sodium alginate, soluble starch, carboxy starch, dextrin, carboxymethyl cellulose, sodium carboxymethyl cellulose, methyl cellulose, ethyl cellulose, hydroxyethyl cellulose, polyvinyl alcohol, polyethylene oxide, polyacrylamide, polyacrylic acid, polyvinylpyrrolidone, carboxyvinyl polymers, polyvinyl ether, maleic acid copolymers, methoxyethylene maleic anhydride copolymers, isobutylene maleic anhydride copolymers, polyethyleneimine, polyvinyl alcohol partially saponified products, hydroxypropylmethyl cellulose, xanthan gum, N-vinylacetamide, and the like. Solubilizer

Examples of the solubilizer include isopropyl myristate, diisopropyl adipate, crotamiton, peppermint oil, N-methyl-2-pyrrolidone, benzyl alcohol, propylene carbonate, and the like. Dye

Examples of the dye which may be used include legal dyes such as FD & C Red No. 2 (amaranth), FD & C Red No. 3 (erythrosine), FD & C Red No. 102 (New Coccine), FD & C Red No. 104 (1) (phloxine B), FD & C Red No. 105 (1) (rose bengal), FD & C Red No. 106 (acid red), FD & C Yellow No. 4 (tartrazine), FD & C Yellow No. 5 (sunset yellow FCF), FD & C Green No. 3 (fast green FCF), FD & C Blue No. 1 (brilliant blue FCF), FD & C Blue No. 2 (indigo carmine), and the like.

### Fragrance

Examples of the fragrance include peppermint oil, cinnamon oil, clove oil, fennel oil, castor oil, turpentine oil, eucalyptus oil, orange oil, lavender oil, lemon oil, rose oil, lemongrass oil and the like, as well as extracts of plants such as rosemary and sage, and the like.

### Ultraviolet Ray Absorbing Agent

Examples of the ultraviolet ray absorbing agent include para-aminobenzoic acid, para-aminobenzoic acid esters, amyl para-dimethylamino benzoate, salicylic acid esters, methyl anthranilate, umbelliferone, esculin, benzyl cinnamate, cinoxate, guaiazulene, urocanic acid, 2-(2-hydroxy-5-methylphenyl)benzotriazole, 4-methoxybenzophenone, 2-hydroxy-4-methoxybenzophenone, dioxybenzone, octabenzone, dihydroxydimethoxybenzophenone, sulisobenzone, benzoresorcinol, octyldimethyl para-aminobenzoate, ethylhexyl para-methoxycinnamate, and the like.

### Inorganic Filler

Examples of the inorganic filler include calcium carbonate, magnesium carbonate, silicic acid salts (for example, aluminum silicate, magnesium silicate and the like), silicic acid, barium sulfate, calcium sulfate, calcium zincate, zinc oxide, titanium oxide, and kaolin.

### pH Adjusting Agent

Examples of the pH adjusting agent include acetic acid, formic acid, lactic acid, tartaric acid, oxalic acid, benzoic acid, glycolic acid, malic acid, citric acid, hydrochloric acid, nitric acid, sulfuric acid, sodium hydroxide, calcium hydroxide, methylamine, ethylamine, propylamine, dimethylamine, diethylamine, dipropylamine, trimethylamine, triethylamine, tripropylamine, monomethanolamine, monoethanolamine, monopropanolamine, dimethanolamine, diethanolamine, dipropanolamine, trimethanolamine, triethanolamine, tripropanolamine, citrate buffers, phosphate buffers, glycine buffers, acetate buffers, and the like.

### Release Sheet

Examples of the release sheet include films of a polyester such as polyethylene terephthalate, polyvinyl chloride, or polyvinylidene chloride, laminate films of wood free paper and polyolefin, and the like. These release sheets are preferably subjected to a silicon treatment on the opposing face to the paste layer, since release of the release sheet is facilitated.

### Production Process

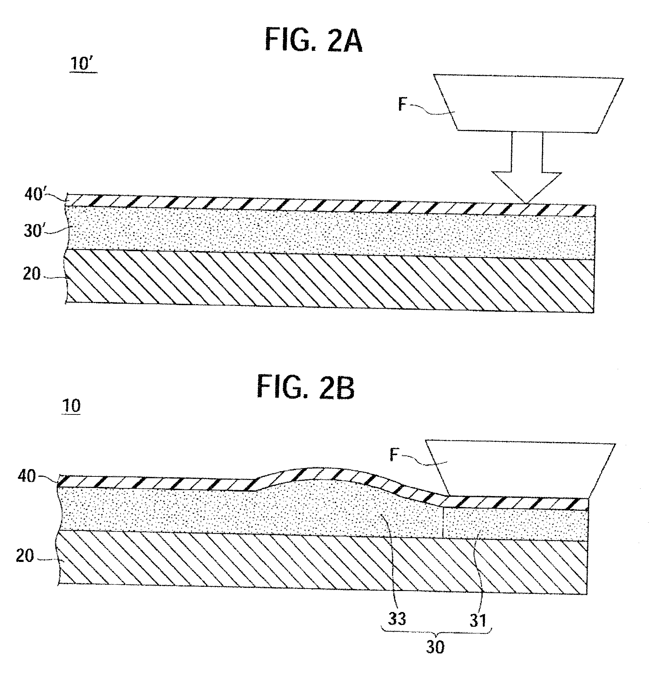

The process for producing the poultice according to the present embodiment is explained with reference to Fig. 2. First, a base (paste material) is prepared according to a common procedure, and a preliminary paste layer is formed by applying this base on a backing or a release sheet. Subsequently, the formed preliminary paste layer is laminated with the release.sheet or backing to produce a complex 10'.

As shown in Fig. 2, a frame F having a shape that meets with edge part 31 of the poultice to be finally formed is pressed against the complex 10', pushed into the frame F, whereby a preliminary edge part 31' and a preliminary central part 33' interposed between the preliminary edge part 31' are formed, in which a thickness of the preliminary edge part 31' is less than a thickness of the preliminary central part 33' (pressing step). Specifically, rotating rollers provided with the frame F on the surface thereof are placed oppositely on the transport track of the composite 10' (not shown in the Figure). When the composite 10' is transported in this state, the frame F is pushed against the composite 10' when the composite 10' passes the rotating rollers.

In the pressing step, the difference between the thickness of the edge part 31 and the thickness of the central part 33 is preferably defined to be no greater than a predetermined value by regulating the force of the pressing (regulating step). The force of the pressing can be regulated by changing the intervals of the frame F and the transport track depending on the thickness of the composite 10'. In other words, increase in the interval of the frame F and the transport track leads to reduction of the force of the pressing, while decrease in the interval leads to elevation of the force of the pressing.

Next, after the composite 10' is cut in the center of preliminary edge part 31', the pieces are stored at a room temperature for several days to permit a crosslinking reaction in the preliminary paste layer 30', whereby a poultice 10 is obtained.

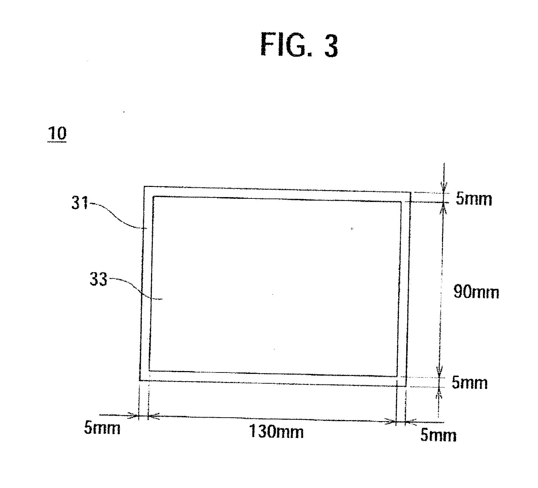

Fig. 3 shows a plan view of the poultice shown in Fig. 1. In the present Embodiment, since the frame F has a grid-like shape of 140 mm x 100 mm with a width of 10 mm, the poultice 10 has a rectangular shape of 140 mm x 100 mm, and the edge part 31 correspond to a part extended from the outer periphery of the poultice 10 inward by no more than 5 mm. It should be noted that the shape and the size of the poultice 10 are not particularly limited thereto.

In the production process, it is preferred that a step of adding the adhesion-enhancing component is not included in the step of preparing the base, in view of capability of producing the poultice easily at a low cost.

### EXAMPLES

### Examples 1 to 6

Pastes were produced according to a common procedure as the formulation shown in Table 1. This paste was laminated on a nonwoven fabric backing, and further covered with a release film, followed by pressing under an appropriate pressure with a frame. Thereafter, thus obtained poultice was stored in a laminate bag, and left to stand for several days in the state in which bags in plural numbers are layered. The poultices after leaving to stand were used in the evaluation tests described below.

**Table 1**

| Ingredient name | Example 1 | Example 2 | Example 3 | Example 4 | Example 5 | Example 6 |
|---|---|---|---|---|---|---|
| Glycol salicylate | 1 | 2 | 2 | | | 1 |
| Rosskastanien extract | 0.1 | | | | | 0.1 |
| Arnica tincture | 1 | 1 | 1 | | | 1 |
| 1-Menthol | 0.3 | 1 | | | 1 | |
| Indomethacin | | | | 0.65 | 1 | |
| Partially neutralized polyacrylic acid "NP-800" | 7 | 7 | 7 | 7 | 7 | |
| Partially neutralized polyacrylic acid "NP-700" | | | | | | 5 |
| Sodium carboxymethyl cellulose | 1 | 1 | 1 | 1 | 1 | |
| Carboxyvinyl polymer | | | | | | 1.5 |
| Gelatin | 1.5 | 1.5 | 1.5 | 1.5 | 1.5 | |
| Polyvinyl alcohol | | | | | | 3 |
| Dry aluminum hydroxide gel | 0.06 | 0.06 | 0.06 | 0.06 | 0.06 | 0.1 |
| Surfactant | 0.2 | 1.6 | 1 | 0.6 | 1.52 | 0.3 |
| Tartaric acid | 0.7 | 0.8 | 0.8 | 0.7 | 0.7 | 0.9 |
| Phosphoric acid | | | | | | 0.02 |
| Sodium edetate | 0.1 | 0.12 | 0.11 | 0.1 | 0.1 | 0.3 |
| Colorant | 0.0003 | | | 0.0015 | | |
| Conc. Glycerin | 35 | 35 | 35 | 35 | 35 | 30 |
| Titanium oxide | 1 | 0.3 | 0.3 | 1 | 1 | 0.3 |
| Silicic anhydride | 1 | 1 | 1 | 1 | 1 | 2 |
| Antiseptic agent | | | | 0.1 | | |
| Crotamiton | | | | 0.6 | 1.1 | |
| Light liquid paraffin | | | | | | 2.4 |
| H-methyl-2-pyxrolidone | | | | 0.5 | 0.8 | |
| Refreshing agent | | | 0.3 | | | 0.3 |
| Fragrance | | | | 0.1 | | |
| Purified water | remainder | remainder | remainder | remainder | remainder | remainder |

| | | | | | | |
|---|---|---|---|---|---|---|
| (numerical values all based on percent by mass) | | | | | | |

### Comparative Examples 1 to 7

Commercially available products were used in Comparative Examples.

Any of the.poultices according to the foregoing Examples and Comparative Examples had a rectangular shape of 140 mm x 100 mm. Each part of these poultices inward by 5 mm from the outer periphery was cut with scissors, whereby a central part corresponding to its inner portion, and an edge part corresponding to the outer portion were separated. The paste adhered to each of these central part and edge part was scraped, and the mass of the paste per unit area was determined by measuring the mass of the scraped paste, and dividing the measurement by each surface area. The results are shown in Table 2.

**Table 2**

| Preparation | Mass of the paste (g) | | |
|---|---|---|---|
| | Central part | Edge part | Edge part/central part x 100 (%) |
| Example 1 | 0.0617 | 0.0597 | 96.68 |
| | 0.0616 | 0.0630 | 102.23 |
| | 0.0611 | 0.0650 | 106.41 |
| | 0.0626 | 0.0644 | 102.93 |
| | 0.0622 | 0.0624 | 100.40 |
| | 0.0621 | 0.0637 | 102.42 |
| | 0.0594 | 0.0602 | 101.31 |
| | 0.0594 | 0.0617 | 103.99 |
| | 0.0610 | 0.0615 | 100.88 |
| Example 2 | 0.0602 | 0.0721 | 119.77 |
| | 0.0610 | 0.0701 | 115.03 |
| | 0.0624 | 0.0681 | 109.20 |
| | 0.0631 | 0.0653 | 103.55 |
| | 0.0632 | 0.0645 | 102.12 |
| | 0.0625 | 0.0654 | 104.63 |
| | 0.0617 | 0.0605 | 98.03 |
| | 0.0622 | 0.0653 | 105.04 |
| | 0.0614 | 0.0659 | 107.29 |
| Example 3 | 0.0620 | 0.0683 | 110.08 |
| | 0.0608 | 0.0698 | 114.92 |
| | 0.0611 | 0.0717 | 117.25 |
| | 0.0595 | 0.0683 | 114.77 |
| | 0.0596 | 0.0697 | 116.96 |
| | 0.0594 | 0.0653 | 109.78 |
| | 0.0576 | 0.0683 | 118.49 |
| | 0.0572 | 0.0680 | 118.96 |
| | 0.0593 | 0.0684 | 115.47 |
| Example 4 | 0.0584 | 0.0691 | 118.28 |
| | 0.0579 | 0.0668 | 115.49 |
| | 0.0584 | 0.0707 | 121.06 |
| | 0.0595 | 0.0706 | 118.59 |
| | 0.0594 | 0.0697 | 117.40 |
| | 0.0594 | 0.0709 | 119.31 |
| | 0.0562 | 0.0677 | 120.46 |
| | 0.0573 | 0.0673 | 117.53 |
| | 0.0572 | 0.0687 | 120.12 |
| Example 5 | 0.0572 | 0.0631 | 110.44 |
| | 0.0565 | 0.0657 | 116.26 |
| | 0.0572 | 0.0662 | 115.71 |
| | 0.0637 | 0.0545 | 85.56 |
| | 0.0634 | 0.0565 | 89.10 |
| | 0.0650 | 0.0554 | 85.16 |
| | 0.0644 | 0.0476 | 73.90 |
| | 0.0628 | 0.0477 | 75.90 |
| | 0.0633 | 0.0496 | 78.27 |
| Example 6 | 0.0856 | 0.0705 | 82.38 |
| | 0.0856 | 0.0717 | 83.79 |
| | 0.0854 | 0.0711 | 83.34 |
| | 0.0813 | 0. 676 | 83.08 |
| | 0.0813 | 0.0716 | 88.00 |
| | 0.0808 | 0.0699 | 86.49 |
| Comparative Example 1 | 0.0866 | 0.0628 | 72.51 |
| | 0.0864 | 0.0641 | 74.14 |
| | 0.0869 | 0.0610 | 70.19 |
| Comparative Example 2 | 0.0904 | 0.0522 | 57.77 |
| | 0.0893 | 0.0528 | 59.15 |
| | 0.0912 | 0.0477 | 52.29 |
| Comparative Example 3 | 0.0855 | 0.0543 | 63.56 |
| | 0.0879 | 0.0477 | 54.33 |
| | 0.0865 | 0.0506 | 58.48 |
| Comparative Example 4 | 0.0889 | 0.0592 | 66.61 |
| | 0.0855 | 0.0520 | 60.76 |
| | 0.0885 | 0.0542 | 61.22 |
| Comparative Example 5 | 0.0898 | 0.0511 | 56.96 |
| | 0.0890 | 0.0514 | 57.73 |
| | 0.0888 | 0.0511 | 57.59 |
| Comparative Example 6 | 0.1175 | 0.0779 | 66.26 |
| | 0.1193 | 0.0642 | 53.31 |
| | 0.1174 | 0.0680 | 57.96 |
| Comparative Example 7 | 0.1133 | 0.0452 | 39.92 |
| | 0.1135 | 0.0497 | 43.75 |
| | 0.1121 | 0.0550 | 49.08 |

As shown in Table 1, any of the poultices of Examples 1 to 6 had the mass of the paste per unit area in the edge part being no less than 70% of the mass of the paste per unit area in the central part. As a matter of fact, runover of the paste layer was not observed in any of the poultices.

### Evaluation 1

First, each of the poultices according to Examples and Comparative Examples was attached to joint of the back of hand of five panelists. Next, each panelist wore a long-sleeve outing shirt, thereby making the edge part of the poultice likely to engage with the sleeve of the shirts. Thereafter, the state of attachment was observed at predetermined times while each panelist kept conducting daily activities. Thus observed state was represented by numerical numbers according to the following criteria. The results are shown in Table 3.

3: Completely stuck without detachment or turning up.

2: Slight detachment of edge part found, but almost completely stuck.

1: Detachment at edge part prominently found.

0: Detached.

**Table 3**

| | Immediately after attachment | 30 min later | 2 hrs later | 4 hrs later | 6 hrs later | 8 hrs later |
|---|---|---|---|---|---|---|
| Example 1 | 3 | 3 | 3 | 3 | 3 | 3 |
| Example 2 | 3 | 3 | 3 | 3 | 3 | 3 |
| Example 3 | 3 | 3 | 3 | 3 | 3 | 3 |
| Example 4 | 3 | 3 | 3 | 3 | 3 | 3 |
| Example 5 | 3 | 3 | 3 | 3 | 3 | 3 |
| Example 6 | 3 | 3 | 3 | 3 | 3 | 3 |
| Comparative Example 1 | 3 | 2 | 2 | 2 | 1 | 1 |
| Comparative Example 2 | 3 | 2 | 2 | 2 | 1 | 1 |
| Comparative Example 3 | 3 | 2 | 2 | 2 | 1 | 1 |
| Comparative Example 4 | 3 | 2 | 2 | 2 | 1 | 1 |
| Comparative Example 5 | 3 | 3 | 3 | 3 | 2 | 1 |
| Comparative Example 6 | 3 | 2 | 2 | 2 | 1 | 1 |
| Comparative Example 7 | 3 | 2 | 2 | 2 | 1 | 1 |

As shown in Table 3, the poultices of Examples 1 to 6 completely stuck even after 8 hours following attaching. In contrast, the poultices of Comparative Examples 1 to 7 started to detach after 6 hours following attaching.

### Example 7

A poultice was produced by a similar procedure to Example 1 except that the paste produced in Example 1 was applied on the backing at a rate of 0.035 g/cm² per unit area.

### Example 8

A poultice was produced by a similar procedure to Example 7 except that the paste was applied on the backing at a rate of 0.05 g/cm² per unit area.

### Example 9

A poultice was produced by a similar procedure to Example 7 except that the paste was applied on the backing at a rate of 0.07 g/cm² per unit area.

### Evaluation 2

The skin patches of Examples 7 to 9 were attached to the lumbar region of five panelists. The state of attachment 12 hours later was observed, and evaluated according to the following criteria. The results are shown in Table 4.

A: Completely stuck.

B: Almost completely stuck although detached in part.

C Almost part detached.

D: Detached.

**Table 4**

| | Example 7 | Example 8 | Example 9 |
|---|---|---|---|
| Panelist 1 | A | A | A |
| Panelist 2 | A | A | A |
| Panelist 3 | A | A | A |
| Panelist 4 | A | B | A |
| Panelist 5 | A | A | A |

As shown in Table 4, any of the skin patches according to Examples 7 to 9 exhibited superior sticking force in spite of the low level of the mass of the paste per unit area being no more than 0.07 g/cm².

## Claims

1. A poultice comprising a backing, and a paste layer, formed of a paste, provided on the backing, wherein:
the paste layer includes an edge part extending from an outer periphery inwardly by no more than 5 mm, and a central part interposed between the edge part;
a mass of the paste per unit area of the paste layer as a whole is no more than 0.10 g/cm²; and
a mass of the paste per unit area in the edge part is no less than 70% of a mass of the paste per unit area in the central part.

2. The poultice according to claim 1, wherein the paste contains a compound represented by formula 1, and does not substantially contain an adhesion-enhancing component that enhances adhesion 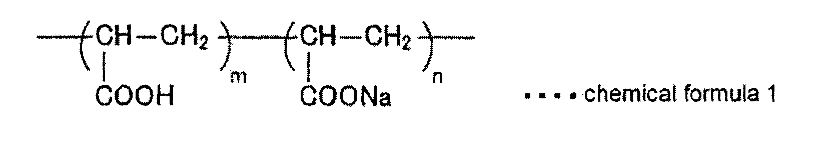 wherein, m/n (molar ratio) is no less than 55/45 and no greater than 75/25.

3. The poultice according to claim 2, wherein the adhesion-enhancing component is at least one selected from the group consisting of polybutene, an alicyclic saturated hydrocarbon resin, a rhodine derivative, a terpene based resin and a phenol based resin.

4. The poultice according to claim 2 or 3, wherein the paste layer does not substantially contain a plasticizer of the adhesion-enhancing component.

5. The poultice according to claim 4, wherein the plasticizer is liquid paraffin.

6. The poultice according to any one of claims 2 to 5, wherein the paste layer has a pH of no lower than 4.50 and no higher than 5.50.

7. The poultice according to any one of claims 1 to 6, wherein the backing has a mass per unit area of no more than 100 g/m².

8. A process for producing a poultice comprising a backing, and a paste layer provided on the backing and formed of a paste,
the process comprising a pressing step of pressing the paste layer at sites to be a peripheral margin to form an edge part and a central part interposed between the edge part, wherein a thickness of the edge part is less than a thickness of the central part, and
wherein the pressing step includes a regulating step of regulating a force of the pressing to make a difference between the thickness of the edge part and the thickness of the central part to be no greater than a predetermined value.
